Europäisches Patentamt

European Patent Office

Office européen des brevets

(19)

(11) Numéro de publication: **0 192 009 B1**

## (12) FASCICULE DE BREVET EUROPEEN

(45) Date de publication de fascicule du brevet:
**04.09.91**

(21) Numéro de dépôt: **85402606.9**

(22) Date de dépôt: **23.12.85**

Demande divisionnaire 90201811.8 déposée le 23/12/85.

(51) Int. Cl.⁵: **C03C 17/23**, C03C 17/25;
C07C 53/06, C07C 51/41,
C03C 23/00, C23C 14/58

(54) **Procédé de prèparation d'un poudre à base de formiate d'indium pour la formation d'une couche mince sur un substrat, notamment en verre.**

(30) Priorité: **22.01.85 FR 8500854**
**03.07.85 FR 8510145**

(43) Date de publication de la demande:
**27.08.86 Bulletin 86/35**

(45) Mention de la délivrance du brevet:
**04.09.91 Bulletin 91/36**

(84) Etats contractants désignés:
**AT BE CH DE FR GB IT LI LU NL SE**

(56) Documents cités:
**EP-A- 0 108 616**
**FR-A- 2 283 098**

**GMELINS HANDBUCH DER ANORGANISCHEN CHEMIE, INDIUM, édition 8, 1936, système no. 37, pages 46, 48, 49, 107; Verlag Chemie GmbH, Weinheim, DE**

(73) Titulaire: **SAINT-GOBAIN VITRAGE INTERNA-TIONAL**
**18, avenue d'Alsace**
**F-92400 Courbevoie(FR)**

(72) Inventeur: **Sauvinet, Vincent**
**149, rue Oberkampf**
**F-75011 Paris(FR)**
Inventeur: **Trouve, Maurice**
**8, rue Gautier 1er**
**F-77140 Nemours(FR)**
Inventeur: **Bletry, Jean**
**11 Ter, rue Mouchy**
**F-78000 Versailles(FR)**
Inventeur: **Bonnaud, Micheline**
**2 Bis, rue Louis Blanc**
**F-92240 Malakoff(FR)**

(74) Mandataire: **Leconte, Jean-Gérard et al**
**Saint-Gobain Recherche 39, Quai Lucien Le-franc**
**F-93304 Aubervilliers Cedex(FR)**

CHEMICAL ABSTRACTS, vol. 81, no. 24, 16 décembre 1974, page 645, réf. No. 16278h; Columbus, Ohio, US. W. Lindel et al.: "Preparation and some properties of indium (III) carboxylates." & Z. ANORG. ALLG. CHEM. 1974, 408 (2), 167-74

INSTRUMENTS AND EXPERIMENTAL TECHNIQUES, vol. 21, no. 6, partie 2, novembre/décembre 1978, juin 1979, pages 1653-1655; Plenum Publ. Corp. Consultants Bureau, New York, US. B.P. KRYZHANOVSKII et al.: "Production of transparent conducting coatings of indium trioxide."

W. Lindel, F. Huber, zeitschrift f r anorg. u. allg. Chemie, 408 (2), 167-179 (1974)

R. E. Mayer, Ann. Chem. Pharm. 150, 145-6, (1869)

P. Pascal, Nouveau Traité de Chimie Minérale, Tome VI, Masson et Cie., Editeurs, Paris, (1961), p. 886-89

**Description**

La présente invention concerne la fabrication de formiate d'indium en poudre.

Le formiate d'indium en poudre est destiné à la formation, par pyrolyse, de couches minces à base d'oxyde d'indium sur un substrat, notamment en verre.

Il est connu de former des couches minces d'oxydes métalliques sur un substrat, notamment en verre, par pyrolyse de poudres de composés métalliques projetés sur ledit substrat, ces couches minces, colorées ou non, ayant essentiellement des propriétés de basse émissivité, de conduction électrique et de transparence.

Pour qu'un composé métallique pulvérulent puisse former un revêtement de bonne qualité sur un substrat, il faut qu'il puisse être distribué régulièrement, qu'il se décompose avec un rendement suffisant et ce, à une température ne dépassant pas 650°C ou 700°C si le substrat à revêtir est en verre et qu'il contienne une proportion de métal suffisante pour conduire à une couche d'oxyde métallique d'épaisseur notable en un court instant (en particulier si ce substrat se déplace rapidement par rapport aux moyens distributeurs de la poudre, comme c'est le cas pour un ruban de verre à la sortie d'un bain dit "float").

On sait déjà réaliser sur des substrats en verre un revêtement d'oxyde d'étain que l'on forme en distribuant sur le substrat, de l'oxyde de dibutylétain en poudre (DBTO) (voir par exemple les publications françaises de brevets FR 2 380 997 et 2 391 966), ou du difluorure de dibutylétain en poudre (DBTF) (voir par exemple le brevet européen 0 039 256 et la publication française de brevet n° 2 542 636).

Ces poudres conduisent certes à des couches satisfaisantes ; cependant, elles sont colorées en réflexion pour les épaisseurs nécessaires à l'obtention de propriétés électroniques intéressantes. Or, la couleur peut ne pas plaire ou ne pas être adaptée au style de la structure environnante. Par ailleurs, de légères variations d'épaisseur de couches entraînent des irrégularités de couleur.

Pour obtenir une parfaite uniformité de la couleur, il faut respecter scrupuleusement certaines conditions, en particulier une qualité constante et bien déterminée de la poudre, et des réglages très précis des installations servant à la distribuer.

La couleur elle-même et les conditions opératoires qu'il est nécessaire de respecter pour qu'elle soit parfaitement uniforme constituent des inconvénients de ces couches à base d'oxyde d'étain.

On a aussi utilisé des composés d'indium, non pas en poudre, mais en solution, en particulier des acétylacétonates d'indium, pour fabriquer des couches minces sur du verre. Mais ces composés sous forme de liquide, ne se pyrolysent qu'a des vitesses insuffisantes. Ils ne conviennent donc pas pour être appliqués sur un ruban de verre défilant à vitesse élevée à la sortie d'un "bain float".

Différents documents ont décrits la préparation du formiate d'indium.

Le document "Zeitschrift für anorganische und allgemeine Chemie", 1974, 408 (2) - W. Lindel et F. Huber (pages 167 à 179) décrit la préparation de carboxylate d'indium, par exemple de formiate d'indium, par action d'acide carboxylique anhydre bouillant sur des copeaux d'indium.

Gmelins Handbuch der anorganischen Chemie", Indium, édition 8, 1936, n° 37 pages 46, 48, 49, 107 et le document "Annalen der chemie und pharmacie" 150, 1869 pages 145-146 R.E. Meyer décrivent la préparation de cristaux de formiate d'indium très solubles, par vaporisation d'une solution d'hydroxyde d'indium dans l'acide formique. Dans le nouveau traité de chimie minérale de Pascal, tome VI, pages 886-889, il est cependant indiqué que de la poudre de formiate d'indium n'a pu être isolée selon la méthode décrit dans les documents précédents.

La présente invention vise à fournir un composé métallique en poudre qui soit susceptible d'être distribuée sur un substrat à température élevée, tel qu'un ruban de verre défilant à la sortie d'un "bain float", en vue de constituer après pyrolyse, une couche de revêtement mince, transparente et électroconductrice. De plus, la pyrolyse de ce composé métallique doit pouvoir s'effectuer à rendement suffisamment élevé pour être compatible avec le défilement rapide du substrat. D'autre part, la couche obtenue à partir de ce composé doit présenter des performances électriques et optiques satisfaisantes pour des épaisseurs correspondant à une couleur neutre en vue d'éviter les inconvénients des couches colorées d'oxyde d'étain de l'art antérieur.

On a trouvé, selon l'invention, un procédé de préparation de formiate d'indium en poudre, convenant pour la formation de telles couches.

L'invention propose un procédé de fabrication du formiate d'indium qui consiste à attaquer l'indium par un acide, puis à précipiter l'hydroxyde d'indium en faisant réagir sur le sel d'indium ainsi obtenu, de l'ammoniaque à une température voisine de l'ébullition, à laver et sécher le précipité et à faire réagir ce dernier avec l'acide formique.

Ainsi, en utilisant de l'acide chlorhydrique comme acide, le procédé de fabrication peut être résumé par les équations suivantes :

$$In + 3HCl \qquad InCl_3 + 3/2 \ H_2$$
$$InCl_3 + 3NH_4OH \qquad In(OH)_3 + 3ClNH_4$$
$$In(OH)_3 + 3HCOOH \qquad In(HCOO)_3 + 3H_2O.$$

Au lieu de l'acide chlorhydrique, on peut aussi bien utiliser l'acide nitrique ou un autre acide.

Le formiate d'indium, comme indiqué précédemment, est utile pour former des couches minces à base d'oxyde d'indium par pyrolyse. Dans ce cas, le formiate d'indium peut être le seul composé métallique de la poudre, mais avantageusement il peut être associé à un ou plusieurs autres composés ayant des températures de décomposition du même ordre que celles du formiate d'indium.

Pour obtenir des couches très conductrices, on peut associer au formiate d'indium des composés d'étain et en particulier DBTO et/ou DBTF qui interviennent alors dans des proportions pondérales pouvant aller jusqu'à 30 %. Ces composés effectuent un dopage cationique de l'oxyde d'indium. En effet, le remplacement d'un atome d'indium par un atome d'étain introduit dans la couche un électron libre. Par ce dopage, on augmente donc la densité des porteurs de charge et la conductivité de la couche.

La poudre de formiate d'indium ou le mélange de poudres peut être projeté sur le substrat, notamment un ruban de verre à l'aide d'une buse, par exemple par l'une des buses décrites dans les demandes de brevets français publiées sous les n° 2 542 636 et 2 542 637 précédée du dispositif de répartition décrit dans la demande de brevet français publiée sous le n° 2 548 556.

Le dopage peut également être effectué avec des composés gazeux, tels que SnCl₄ ou des organo-stanniques gazeux, tels que BuSnCl₃. Dans ce cas, le dopant est mélangé avec le gaz dans lequel la poudre est en suspension, et/ou avec les gaz qui servent à l'accélération et/ou à l'homogénéisation de la poudre. Le dopant peut encore être aspiré par la buse dans la chambre de contrôle décrite dans la demande de brevet français publiée sous le n° 2 542 636 et/ou encore être amené à la sortie de la buse de projection par une conduite spéciale.

Avantageusement après dépôt et formation de la couche d'oxyde métallique sur le substrat, celle-ci subit un traitement thermique.

Par ce traitement généralement on cherche à augmenter le nombre de lacunes d'oxygène de la couche de façon à abaisser l'émissivité de ladite couche, donc à améliorer ses propriétés de basse émissivité. Mais on peut aussi, au contraire diminuer le nombre de lacunes d'oxygène de la couche de façon à augmenter l'émissivité de ladite couche donc à amoindrir ses propriétés de basse émissivité qu'elle possédait préalablement.

Suivant un premier mode de réalisation ce traitement thermique est constitué par un séjour du substrat revêtu dans une enceinte chauffée, sous atmosphère contrôlée, séjour qui est plus ou moins long, qui dépend de la température, du caractère réducteur de l'atmosphère, et qui peut s'échelonner de quelques secondes à plusieurs heures.

L'amélioration des propriétés de basse émissivité demandera un traitement thermique en atmosphère réductrice ou éventuellement neutre.

Dans le cas d'une atmosphère réductrice, celle-ci peut être constituée par une atmosphère de composition identique à celle du "bain float", à savoir 90% d'azote et 10% d'hydrogène.

Par ce traitement en atmosphère réductrice ou neutre, on augmente la concentration en lacunes d'oxygène dans la couche, ce qui entraîne un dopage anionique favorable à l'augmentation de la conduction électrique et de la réflexion infrarouge de la couche. Selon la durée et la température du traitement, on peut faire varier considérablement la densité des porteurs de charges, par exemple de 1.1020 à 20.1020 porteurs par cm³ et leur mobilité, par exemple de 10 à 50cm.V⁻¹.s⁻¹.

Ce traitement peut être pratiqué immédiatement après la formation de la couche d'oxyde, directement sur la ligne de fabrication du verre, par exemple dans une étenderie de recuisson, de manière à profiter de la chaleur du verre.

Il peut être également pratiqué plus tard sur une installation séparée de la ligne de fabrication du verre (arche de recuisson annexe, ligne de trempe, de bombage).

Dans ce cas, on réchauffe le substrat revêtu jusqu'à une température d'au moins 300°C compatible avec le substrat, on le maintient en atmosphère réductrice ou neutre à cette température élevée pendant un temps d'autant plus court que la température est plus élevée et que l'atmosphère est plus réductrice (ce temps de maintien pouvant s'échelonner de quelques secondes à plusieurs heures), puis on le refroidit sous atmosphère réductrice ou neutre au moins jusqu'à une température où le contact avec une atmosphère oxydante n'altère plus les performances de la couche.

Avantageusement, le refroidissement contrôlé sous atmosphère réductrice ou neutre est pratiqué jusqu'à une température qui est au maximum de l'ordre de 300°C. Dans le cas de traitement simultané à la trempe, les buses de soufflage du gaz de trempe sont alimentées par le gaz réducteur ou neutre nécessaire au traitement.

En résumé, l'efficacité du traitement de la couche augmente avec :

. le caractère réducteur de l'atmosphère de traitement, par exemple avec le pourcentage d'hydrogène d'un mélange $N_2$ + X % $H_2$ où X, est positif ou nul, notamment X = 10 ou X = 0,

. la température, celle-ci étant comprise entre 300 et 650°C,

. le temps, qui peut varier de quelques secondes à quelques heures.

Suivant un second mode de réalisation, ce traitement thermique est obtenu par un chauffage intense en atmosphère contrôlée, réductrice ou au contraire oxydante suivant la finalité recherchée, pendant un temps inférieur à la seconde. Matériellement cela est obtenu en soumettant le substrat revêtu de la couche à traiter à l'action d'au moins une flamme, réductrice ou au contraire oxydante suivant qu l'on désire, augmenter ou au contraire amoindrir les propriétés de basse émissivité de la couche ainsi traitée, réduire l'oxyde en métal ou au contraire oxyder le métal.

Ce traitement est alors avantageusement réalisé par défilement du substrat revêtu dans la flamme d'au moins un brûleur alimenté en gaz dont les caractéristiques réductrices ou oxydantes sont contrôlées et adaptées à la nature réductrice ou oxydante souhaitée dudit traitement.

Il s'agit donc d'un traitement immédiat (temps de traitement inférieur à la seconde), ne nécessitant qu'une installation réduite, simple, peu coûteuse et facile à conduire.

L'échauffement du substrat à la flamme peut être faible (inférieure à 70°C) dans la mesure où la flamme est disposée par rapport audit substrat du côté du revêtement, si bien que le niveau de trempe d'un substrat en verre préalablement trempé est conservé, ou que des substrats sensibles à la chaleur, en matériau autre que le verre ou comportant en association avec le verre d'autres matériaux tels que le polyvinylbutytal (PVB), ne sont pas altérés.

Le ou les brûleurs nécessaires au traitement de la couche sont alimentés en un mélange de gaz comburant (oxygène ou mélange de gaz neutre et d'oxygène) et de gaz combustible contenant de l'hydrogène et/ou du carbone (hydrogène, monoxyde de carbone, alcane, alcène ou alcyne gazeux).

Les proportions de comburant et de combustibles sont telles que le mélange n'est pas shoechiométrique mais contient au contraire soit un excès de gaz réducteur, soit un excès de gaz oxydant, suivant que l'on désire effectuer un traitement réducteur ou un traitement oxydant.

Les brûleurs utilisables sont, de préférence pour des raisons de sécurité et de facilité d'emploi, des brûleurs linéaires à mélange externe.

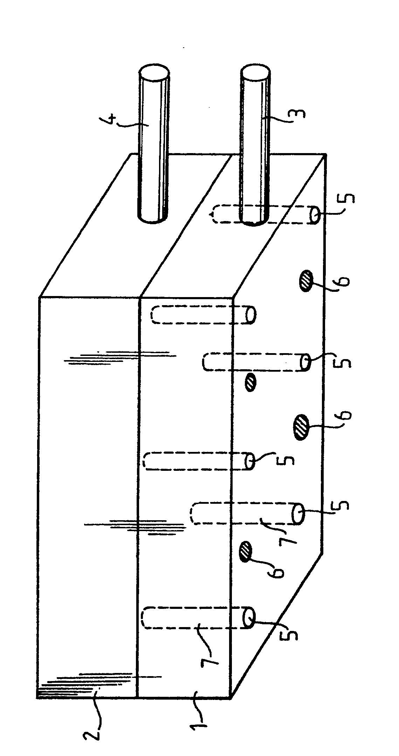

Ces brûleurs sont du type illustré par la figure jointe, c'est-à-dire qu'ils possèdent deux chambres 1 et 2 d'amenée des gaz combustible et comburant, elles mêmes alimentées par les tuyauteries 3 et 4. Chacune de ces chambres 1 et 2 débouche à l'extérieur du brûleur par des trous 5 et 6, les trous 5 délivrant le gaz de la chambre supérieure 2 étant reliés à cette chambre 2 par des conduites 7 qui traversent de façon étanche la chambre inférieure 1. Pour un bon mélange des gaz, les trous 5 et 6 sont disposés en quinconce. Dans La mesure où de grandes longueurs de brûleurs sont nécessaires, des déflecteurs non figurés, ou une forme en biseau des chambres 1 et 2 peuvent être prévus pour assurer une égalité de distribution des gaz d'un bout à l'autre du brûleur.

Des brûleurs de ce type sont distribués par la Société française "AIR LIQUIDE" sous la dénomination "brûleurs FMT".

La couche d'oxyde obtenue sur un substrat chauffé, par projection d'une poudre de formiate d'indium, associée ou non à des composés d'étain aptes à agir comme dopants vis à vis de l'indium, tels que l'oxyde de dibutyl étain (DBTO), et/ou le difluorure de dibutyl étain (DBTF), est par fabrication trop oxydée pour posséder de bonnes caractéristiques de basse émissivité et de basse résistivité. On peut lui faire subir le traitement de "brûlage" selon l'invention, de façon à améliorer ses caractéristiques. Le réglage des débits et des proportions de gaz oxydant et réducteur dépend de la nature des gaz, de l'installation et des conditions opératoires, et est fonction de l'émissivité et de la résistivité désirées pour la couche.

Il est possible de déterminer expérimentalement pour une installation donnée et pour des conditions opératoires données (vitesse de défilement du substrat, distance vis-à-vis des brûleurs) les réglages de débit de gaz de façon à établir les débits limites pour réduire totalement la couche à l'état métallique, l'apparition de cet état métallique se reconnaissant à ce que la couche change d'aspect, acquiert une certaine réflectivité dans le visible qui peut aller jusqu'à la réflectivité d'un miroir et peut dans certains cas, en particulier pour l'oxyde d'indium transformé, devenir peu adhérente au substrat.

Pour cela on règle les proportions respectives de gaz oxydant et de gaz réducteur de façon à disposer d'un mélange légèrement plus réducteur que le mélange stoechiométrique, soit 10 % environ en plus de gaz réducteur par rapport au débit correspondant au mélange stoeohiométrique. Ensuite on pratique le traitement à la flamme de la couche sur divers échantillons en augmentant progressivement le débit global du mélange jusqu'à obtenir la réduction de la couche à l'état métallique. Ce réglage de débits limites étant obtenu, on sait que les réglages permettant d'atteindre les niveaux de basse émissivité et de basse résistivité désirés s'obtiendront en diminuant le débit de gaz réducteur par rapport au débit limite préalablement déterminé.

Grâce à cette technique d'échauffement rapide en atmosphère contrôlée, on a pu obtenir les propriétés optimales de basse émissivité et de basse résistivité d'une couche à base d'oxyde d'indium, déposée par pyrolyse de poudre comme indiqué précédemment, en faisant défiler le substrat revêtu de sa couche, à 1 cm sous un brûleur linéaire à mélange externe, ayant comme longueur la largeur de la couche à traiter, à une vitesse de 3 cm/s, le débit d'oxygène étant de 1,9 1 par minute et par centimètre de longueur de brûleur, le débit d'hydrogène étant de 4 l/min.cm.

En variant la vitesse de défilement d'un même substrat et en la portant à 5 cm/s, on a eu besoin d'un débit d'oxygène de 2,3 l/min.cm. et un débit d'hydrogène de 4,7 l/min.cm.

En augmentant encore la vitesse de défilement de façon à ce qu'elle atteigne la vitesse de défilement du ruban de verre dans les installations "float" par exemple 20 cm/s, on a eu besoin pour obtenir les performances maximales de la couche, de l'ordre de 6 l/min.cm d'oxygène et 13 l/min.cm d'hydrogène.

Avec l'un ou l'autre de ces traitements thermiques on a pu traiter des couches obtenues par pyrolyse d'une poudre de formiate d'indium et de 4% en poids de DBTO et obtenir les résultats énumérés dans les exemples qui suivent :

EXEMPLE I

| | Avant traitement | Après traitement |
|---|---|---|
| épaisseur (Angstroems) | 900 | 900 |
| Coef. moyen de réflexion IR | 0,33 | 0,70 |
| Emissivité | 0,67 | 0,30 |
| Facteur énergétique de transmission (%) | 74,5 | 76,0 |
| Facteur de transmission lumineuse (%) | 74,3 | 79,0 |
| Résistance d'un carré (ohms) | 205 | 35 |

Pour obtenir une telle couche on a utilisé une poudre à base de formiate d'indium mélangée avec 4% en poids de DBTO, qu'on a distribuée sur un ruban de verre défilant à 18m/min, dont la température de surface était de 600° C. Après pyrolyse de la poudre, on a soumis le substrat en verre ainsi revêtu à un traitement thermique consistant en une recuisson dans une enceinte à 600° C pendant 2 min sous atmosphère d'azote puis en un refroidissement progressif jusqu'à 300° C, toujours sous atmosphère non oxydante, d'azote pendant 2 min.

EXEMPLE II

|  | : Avant traitement | : Après traitement : |
| --- | --- | --- |
| épaisseur (Angstroems) | : 1900 | : 1900 : |
| Coef. moyen de réflexion IR | : 0,53 | : 0,87 : |
| Emissivité | : 0,47 | : 0,13 : |
| Facteur énergétique de transmission (%) | : 77 | : 72 : |
| Facteur de transmission lumineuse (%) | : 81,3 | : 83,0 : |
| Résistance d'un carré (ohms) | : 92 | : 11 : |

EXEMPLE III

Dans les mêmes conditions que pour les exemples I et II, sauf en ce qui concerne le débit de poudre distribué sur le substrat en verre, on a réalisé une couche rouge mauve en réflexion, légèrement verte en transmission, dont les caractéristiques avant et après traitement thermique en enceinte sous atmosphère non oxydante sont les suivants :

|  | : Avant traitement | : Après traitement : |
| --- | --- | --- |
| épaisseur (Angstroems) | : 3000 | : 3000 : |
| Coef. moyen de réflexion IR | : 0,76 | : 0,89 : |
| Emissivité | : 0,24 | : 0,11 : |
| Facteur énergétique de transmission (%) | : 78,8 | : 66,0 : |
| Facteur de transmission lumineuse (%) | : 85,8 | : 82 : |
| Résistance d'un carré (ohms) | : 25 | : 7,5 : |

EXEMPLE IV

On a soumis la même couche que celle de l'exemple II, puis de l'exemple III, avant traitement thermique, à la flamme d'un brûleur alimenté en un mélange d'oxygène et d'hydrogène comme décrit précédemment. Quelle que soit la vitesse de défilement sous la flamme du substrat revêtu, avec bien entendu des débits de gaz adaptés, on a obtenu après traitement, des couches ayant les mêmes caractéristiques que celles obtenues après traitement, respectivement dans les exemples II et III.

De la même façon que le premier type de traitement thermique, ce traitement par échauffement intense mais court, par exemple dans la flamme d'un brûleur, peut être pratiqué immédiatement après la fabrication

7

et le revêtement du verte, par exemple sur la ligne float même, mais il peut tout aussi bien être réalisé plus tard. C'est d'ailleurs ce qui est inévitable lorsque le verre doit être trempé ou feuilleté. Dans ce cas, le verre revêtu est découpé, trempé ou feuilleté, puis traité thermiquement.

Ainsi donc, avec l'un ou l'autre des deux traitements thermiques proposés, on a pu obtenir des couches à base de formiate d'indium et d'un ajout de DBTO ou de DBTF, ayant des résistivités électriques de l'ordre de 2 à 10$^{-4}$ cm. On a pu également en modulant l'intensité du traitement thermique, obtenir des performances des couches, intermédiaires entre les performances de la couche non traitée et des performances optimales mesurées sur une couche ayant subi le traitement le plus intense.

Avec de tels traitements thermiques on peut aussi, au lieu d'abaisser la résistivité et l'émissivité d'une couche, au contraire l'augmenter. Ainsi, à partir d'un substrat revêtu ayant une résistance carré de 15 ohms, on a pu atteindre une résistance de 16 ohms, ou de 2000 ohms en faisant défiler le substrat à 1 cm sous le brûleur, respectivement à 10 cm/s et à 6 cm/s, le débit d'oxygène dans les deux cas étant de 2,4 l/min.cm et celui d'hydrogène étant de 3 l/min.cm. Un tel traitement conduisant à l'abaissement de la résistivité et de l'émissivité est bien entendu également possible avec le premier mode de traitement thermique, il suffit de prévoir une atmosphère contrôlée non plus réductrice, mais oxydante.

On peut également avec l'un ou l'autre des deux modes de traitement thermique proposés, créer sur un même vitrage revêtu des zones de conductions électriques différentes par application de traitements thermiques différents. Ce genre de traitements thermiques différenciés suivant les zones d'un même vitrage est particulièrement aisé quand on met en oeuvre le second type de traitement thermique. Pour cela, on modifie localement ou momentanément les conditions d'exposition de la couche aux moyens de chauffage.

Dans cet ordre d'idée, on fait varier la vitesse de défilement du substrat revêtu à traiter par rapport au brûleur en fonction du résultat désiré. Avec un brûleur disposé transversalement par rapport à la direction de défilement du substrat, en modulant la vitesse de défilement dudit substrat, on a obtenu des couches à bandes transversales ayant des propriétés différenciées.

On a pu obtenir aussi des bandes longitudinales dans le sens de déplacement du substrat, à caractéristiques électriques et optiques différentes en ajoutant face à ces zones des brûleurs supplémentaires ou en effectuant le traitement sur toute la largeur du substrat à l'aide d'une pluralité de brûleurs réglés différemment les uns des autres.

Ces traitements thermiques et en particulier celui par brûleurs sont donc particulièrement adaptés à la fabrication de vitrages à couches ayant des zones à propriétés électriques, thermiques, optiques, différenciées. Ainsi, par exemple, des vitrages chauffants à couches minces possédant des résistances plus ou moins importantes suivant les zones pourront être fabriqués.

Le second mode de traitement thermique proposé, autorise comme déjà dit la fabrication de vitrages trempés ou feuilletés à couches minces.

En effet, alors que le premier mode de traitement thermique proposé altère la trempe, empêche le traitement de feuilletés ou de substrats non résistants à la température, le second mode de traitement thermique proposé est si bref que le substrat n'a pas le temps d'être échauffé, en particulier si le moyen de chauffage est disposé du côté de la couche. En conséquence les intercalaires en P.V.B. (polyvinylbutyral) ou autres, des vitrages feuilletés, les substrats non résistants à la chaleur, les contraintes obtenues par la trempe, sont conservés sans altération.

Ce second mode de traitement thermique est donc particulièrement adapté à la fabrication de vitrages trempés à couches minces, le niveau de trempe étant élevé et autorisant en particulier l'usage de tels vitrages en tant que vitrages pour automobiles (c'est-à-dire avec une fragmentation telle que définie dans le règlement 43 des Nations Unies pour l'homologation des vitrages automobiles) le niveau d'émissivité et de résistivité étant également de bonne qualité, c'est-à-dire une émissivité inférieure à 0,15 et une résistivité inférieure à 3. 10$^{-4}$ ohm.cm.

Il est donc également particulièrement adapté à la fabrication de vitrages feuilletés comportant au moins une feuille de verre revêtue d'une couche mince nécessitant un traitement thermique pour acquérir ses caractéristiques définitives.

Ce second mode de traitement thermique a été décrit en utilisant un ou plus brûleurs à gaz, remarquables pour leur simplicité, mais d'autres moyens de chauffage aptes à fournir en moins d'une seconde une énergie élevée peuvent aussi être utilisés ; ainsi par exemple une torche à plasma micro-onde alimentée en gaz réducteur ou au contraire oxydant suivant que l'on désire, comme déjà dit, soit réduire l'oxyde ou abaisser l'émissivité et la résistivité de la couche, soit au contraire oxyder le métal ou augmenter l'émissivité et la résistivité de la couche.

L'invention a été décrite en prenant des vitrages, destinés par exemple aux bâtiments (vitrages bas émissifs, vitrages chauffants, et en général vitrages utilisant les propriétés optiques et/ou électriques des couches), ou à l'automobile (vitrages chauffants, vitrages de sécurité utilisant les propriétés de conduction de la couche pour détecter une effraction, vitrages antenne...), mais elle peut aussi trouver une application intéressante dans le revêtements de substrats divers, isolants en particulier, articles en verre tels que bouteilles, flacons, verreries diverses, éléments d'optique, lampes d'éclairage, fibre de verre, ou encore articles en silice, matériaux réfractaires, alumine.

Le matériel utilisé pour projeter la poudre ou le mélange de poudres sur des objets séparés est alors généralement différent des buses auxquelles on s'est référé ci-dessus. On utilisera par exemple des pistolets classiques de pulvérisation de poudres.

La description ci-dessus a exclusivement concerné des composés en poudre, à base de formiate d'indium et leur emploi sous forme de poudre.

Cependant, ces poudres à base de formiate d'indium peuvent également être transformées et être employées en phase autre que solide et pulvérulente.

En particulier, la poudre peut être mise en solution, notamment dans du méthanol et le composé à base de formiate d'indium en solution, peut être distribué sur un substrat, y être pyrolysé et former ainsi une couche d'oxyde métallique qui pourra subir un traitement thermique apte à modifier les caractéristiques de la couche initiale.

Les traitements thermiques pratiqués postérieurement au dépôt de la couche ont été décrits comme étant appliqués à une couche d'oxyde d'indium dopée ou non à l'étain obtenue par pyrolyse d'une poudre à base de formiate d'indium ; mais ils peuvent également s'appliquer avec succès à toute couche à base d'oxyde d'indium, dopée ou non, à l'étain ou autre, obtenue par une autre voie, par exemple pyrolyse liquide, CVD, ou technique sous vide, que le composé de départ soit du formiate ou autre.

Ces traitements thermiques peuvent même s'appliquer à toute autre couche métallique qui comme la couche à base d'oxyde d'indium est non stoechiométrique, ainsi par exemple les couches à base d'oxyde de vanadium, de Zn, de Sn...

Ces traitements thermiques peuvent même permettre d'obtenir, à partir de couches d'oxyde métallique, des couches de métal. L'obtention d'une couche de métal à partir d'une couche d'oxyde a été décrite comme une phase du réglage des brûleurs servant aux traitements thermiques, mais l'obtention d'une telle couche de métal peut être le but final.

Les traitements thermiques peuvent également être mis en oeuvre pour recristalliser une couche mal cristallisée ou amorphe, en particulier pour modifier ses propriétés électroniques.

## Revendications

1. Procédé de préparation de formiate d'indium en poudre, caractérisé en ce qu'il consiste à attaquer l'indium par un acide, puis à précipiter l'hydroxyde d'indium en faisant réagir sur le sel d'indium ainsi obtenu, de l'ammoniaque à une température voisine de l'ébullition, à laver, à sécher le précipité et à faire réagir ce dernier avec l'acide formique.

2. Procédé conforme à la revendication 1, caractérisé en ce qu'on utilise de l'acide chlorhydrique ou de l'acide nitrique.

## Claims

1. Process for the preparation of indium formate in powder form, characterized in that it consists of attacking the indium with an acid, then precipitating the indium hydroxide by causing ammonia at a temperature close to boiling point to react on the indium salt thus obtained, of washing, of drying the precipitate and of causing the latter to react with formic acid.

2. Process according to Claim 1, characterized in that hydrochloric acid or nitric acid is used.

## Patentansprüche

1. Verfahren zur Herstellung von Indiumformiat in Pulverform, dadurch gekennzeichnet, daß es in dem Einwirkenlassen einer Säure auf das Indium, danach der Ausfällung von Indiumhydroxid durch Einwirkenlassen von Ammoniak auf das so erhaltene Indiumsalz bei einer Temperatur in der Nähe der Siedetemperatur, der Wäsche, der Trocknung des Niederschlags und der Umsetzung von letzterem mit Ameisensäure besteht.

2. Verfahren nach Anspruch 1, dadurch gekennzeichnet, daß man Chlorwasserstoffsäure oder Salpeter-

säure einsetzt.